# EUROPÄISCHE PATENTSCHRIFT

(11) **EP 3 955 810 B1**
(45) Veröffentlichungstag und Bekanntmachung des Hinweises auf die Patenterteilung: **24.06.2026**
(21) Anmeldenummer: 20718524.0
(22) Anmeldetag: 02.04.2020
(51) Int. Cl.: A61B 5/274, A61B 5/291, A61B 5/296

(54) **ELEKTRODE**
ELECTRODE
ÉLECTRODE

(30) Priorität: 16.04.2019 AT 503432019
(43) Veröffentlichungstag der Anmeldung: 23.02.2022
(73) Patentinhaber: Leonhard Lang GmbH, 6020 Innsbruck (AT)
(72) Erfinder: KUPSA, Thomas, 6020 Innsbruck (AT)
(74) Vertreter: Torggler & Hofmann Patentanwälte - Innsbruck
(86) Internationale Anmeldenummer: PCT/AT2020/060136
(87) Internationale Veröffentlichungsnummer: WO 2020/210849

(56) Entgegenhaltungen:
- WO-A2-01/17423
- WO-A2-2018/071944
- US-A- 5 150 708
- US-A1- 2011 295 100

## Beschreibung

Die Erfindung betrifft eine Elektrode nach Oberbegriff des Anspruchs 1. Weiters betrifft die Erfindung ein Verfahren zum Herstellen einer solchen Elektrode.

Derartige medizinische Hautelektroden können als Messelektroden eingesetzt werden, die elektrische Signale vom menschlichen Körper ableiten. Sie können aber auch als Therapieelektroden eingesetzt werden, um Ströme dem menschlichen Körper zuzuführen. Die Elektroden werden zu diesem Zweck auf die Haut aufgeklebt und verfügen auf ihrer Unterseite im Allgemeinen über ein elektrisch leitendes Gel oder ein anderes elektrisches Kontaktmedium, das galvanisch mit einem Anschlusselement der Elektrode in Kontakt steht. An diesem Anschlusselement kann ein elektrischer Signalleiter angeschlossen werden, über den Ströme aus der Elektrode abgeleitet oder der Elektrode zugeführt werden können.

Ein Typ von Elektroden weist an der der Haut abgewandten Oberseite ein vorstehendes elektrisch leitendes Anschlusselement mit einer meist im Wesentlichen kugelkopfförmigen Anschlussstelle auf, an die sich ein Hals anschließt.

Bei der bisherigen Konstruktion von Elektroden dieses Typs war das Anschlusselement zweiteilig ausgeführt. Der obere Teil (Oberknopf, Stud) dient als Kontakt- und Ankerelement für handelsübliche Signalleiter, beispielsweise EKG-Leitungen. Im Wesentlichen unterhalb des Trägers, also auf der der Haut zugewandten Seite, ist ein Unterknopf (Eyelet) der der Übernahme elektrischer Potenziale direkt vom Gel (Kontaktmedium) oder zur Übergabe an das Gel dient. Dabei ist das Eyelet sowohl elektrisch wie mechanisch mit dem Stud verbunden und zwar im Allgemeinen durch Vernieten der beiden Teile, derart, dass zwischen einem flanschartig seitlich abstehenden Haltebereich des Studs und einem ebensolchen Haltebereich des Eyelets das Trägermaterial der Elektrode fest eingeklemmt ist. Eine solche Konstruktion bietet einerseits einen guten mechanischen Halt des Anschlusselementes am Träger der Elektrode und erlaubt es andererseits, das Eyelet aus Materialien zu fertigen, die für eine Signalelektrode elektrische günstige Eigenschaften aufweisen, beispielsweise kann es dazu mit Silber beschichtet sein, wobei die Silberschicht wiederum ganzflächig oder zumindest in einem Teilbereich, der mit dem Gel in Kontakt steht, mit einer Schicht aus Silber / Silberchlorid (Ag /AgCl) überdeckt ist. Es besteht auch die Möglichkeit, dass das Eyelet das Gel nicht direkt berührt. Dann ist bei diesen sogenannten Off-Center-Elektroden ein Querleiter vorgesehen, der das Eyelet mit dem Gel verbindet.

Die Elektroden nach dem Stand der Technik sind allerdings teuer - wobei bei derartigen Massenartikeln bereits geringfügige Preisunterschiede ins Gewicht fallen.

Die anmeldereigene Patentschrift AT 519280 A1 /WO 2018/071944 A2 schlägt deshalb vor, anstatt einer häufig üblichen zweiteilige Konstruktion, bei der das Anschlusselement aus zwei miteinander vernieteten Teilen (Stud und Eyelet) besteht, als Anschlusselement nunmehr einen einzigen Teil vorzusehen, der einerseits die Anschlussstelle zum lösbaren Anschließen eines Signalleiters bereitstellt und andererseits mit dem elektrischen Querleiter (vorzugsweise galvanisch) in Verbindung steht.

Auch die Patentschriften WO 01/17423 A2 und US 5,150,708 A offenbaren Elektroden mit solchen einteilig ausgebildeten Anschlusselementen.

Eine solche einteilige Ausbildung des Anschlusselements weist allerdings immer noch einige Nachteile auf.

Beispielsweise wird eine zusätzliche Stützlage benötigt, um das Anschlusselement am Träger ausreichend zu befestigen. Dies wirkt sich einerseits auf den Materialbedarf für eine Elektrode aus, als auch auf die Dauer der Fertigung einer Elektrode.

Der Materialbedarf und die Dauer der Fertigung einer Elektrode sind allerdings maßgebend für die Fertigungskosten einer solchen Elektrode. Es ist deshalb wünschenswert, den Materialbedarf sowie die Dauer der Fertigung einer Elektrode möglichst gering zu halten.

Aufgabe der Erfindung ist es daher, eine gegenüber der in der Patentschrift AT 519280 A1 geoffenbarten Elektrode - insbesondere in Hinblick auf Fertigungskosten und -dauer - verbesserte Elektrode sowie ein Verfahren zur Herstellung einer solchen Elektrode anzugeben.

Erfindungsgemäß wird dies durch eine Elektrode gemäß Anspruch 1 und einem Verfahren gemäß Anspruch 14 erreicht.

Es ist also vorgesehen, dass das Anschlusselement zumindest einen durch den Träger reichenden Vorsprung aufweist, der an seinem Ende einen durch Verformung gebildeten, erweiterten Bereich aufweist, wobei durch diesen verformten, erweiterten Bereich einerseits eine elektrische Verbindung zwischen dem Leiter und dem Anschlusselement und andererseits eine mechanische Befestigung des Anschlusselements am Träger herstellbar sind.

Dadurch kann sowohl der Materialbedarf als auch die Fertigungsdauer einer Elektrode reduziert werden.

Vorteilhaft ist auch, dass das Anschlusselement durch den durch Verformung gebildeten, erweiterten Bereich sowohl form- als auch kraftschlüssig mit dem Träger und/oder dem Leiter verbunden ist.

Besonders bevorzugt kann vorgesehen sein, dass das Anschlusselement aus einem einzigen Teil besteht, das die Anschlussstelle zum lösbaren Anschließen einer Signalleitung aufweist.

Das Anschlusselement selbst kann durchaus aus mehreren Materialien bestehen, zum Beispiel aus vernickeltem Messing oder einem mit leitfähigen Material (insbesondere Kohlenstofffasern) dotiertem Kunststoff.

Besonders bevorzugt ist eine Ausbildung des Anschlusselementes, bei dem dieses derart ausgebildet ist, dass das Anschlusselement einen im Wesentlichen kugelförmigen Kopf, einen daran anschließenden, im Durchmesser reduzierten Hals, einen an das Ende des Halses anschließenden flanschförmig seitlich abstehenden Haltebereich und zumindest einen an den Haltebereich anschließenden Vorsprung aufweist.

Der Vorsprung wird (vorzugsweise ohne seitliche Berührung) durch eine Öffnung im Träger geführt, während der flanschförmig seitlich abstehende Haltebereich auf der Oberseite des Trägers aufliegt. Der im Durchmesser erweiterte, flanschförmig seitlich abstehende Haltebereich hält das Anschlusselement auch bei hohen Druckbelastungen sicher am Trägermaterial fest.

Der verformte, erweiterte Bereich des Vorsprungs des Haltelements liegt an der der Haut zugewandten Unterseite des Trägers bzw. an dem Leiter an und gewährleistet somit auch bei Druckbelastungen auf das Anschlusselement einen guten Halt des Anschlusselements am Träger.

Es kann auch vorgesehen sein, dass der zumindest eine Vorsprung aus zumindest einem ersten Segment und zumindest einem zweiten Segment besteht, wobei sich beide Segmente in einer Ausgangslage in einer horizontalen Lage oder in einer vertikalen Lage befinden, oder wobei sich zumindest eines der zumindest zwei Segmente in einer horizontalen Lage und zumindest ein zweites der zumindest zwei Segmente in einer vertikalen Lage befindet, und dass der Haltebereich durch zumindest eines der zumindest zwei Segmente gebildet wird.

Bei diesem Ausführungsbeispiel der Erfindung kann die Verformung des verformbaren Bereichs durch ein einfaches Umbiegen zumindest eines der zumindest zwei Segmente erfolgen.

Gemäß einem weiteren Ausführungsbeispiel der Erfindung kann vorgesehen sein, dass der seitlich abstehende flanschartige Haltebereich aus zumindest zwei Flügelsegmente besteht, wobei die zumindest zwei Flügelsegmente einen gegenüber einer horizontalen Lage geneigten Abschnitt aufweisen, und dass der Vorsprung durch die zumindest zwei Flügelsegmente gebildet wird, wobei vorzugsweise die zumindest zwei Flügelsegmente zumindest abschnittsweise scharfkantig ausgebildet sind.

Die zumindest zwei Flügelsegmente können dabei kongruent oder nicht kongruent ausgebildet sein. Im letzteren Fall können insbesondere die geneigten Abschnitte unterschiedlich lang ausgebildet sein.

Bei einem solchen Ausführungsbeispiel ist es möglich, das Anschlusselement ohne vorherigem Herstellen einer durchgehenden Öffnung durch den Träger und den Leiter - durch Kombination einer Dreh- und Druckbewegung des Anschlusselements auf der Oberseite des Leiters - in den Träger bzw. Leiter einzubringen. Zumindest einer der zumindest zwei geneigten Abschnitte kann durch einfaches Biegen in Richtung der Unterseite des Trägers umgeformt werden.

Sind die Flügelsegmentabschnitte scharfkantig ausgebildet, so erleichtert dies ein Durchdringen eines Trägers und eines Leiters.

Eine weitere Ausführungsform der Erfindung sieht vor, dass der zumindest eine Vorsprung als sich in eine dem Haltebereich entgegengesetzte Richtung verjüngender Dorn ausgebildet ist. Dadurch ist es möglich, das Anschlusselement ohne vorheriges Herstellen einer durchgehenden Öffnung durch den Leiter und den Träger in den Träger bzw. Leiter einzubringen. Es wird also ein Arbeitsschritt eingespart.

An die elektrischen Eigenschaften des Anschlusselements sind beim Erfindungsgegenstand keine hohen Anforderungen gestellt. Er kann daher aus kostengünstigem Material, beispielsweise aus einem einfachen Metallblech bestehen. Das Anschlusselement braucht nämlich keine besonderen elektrischen Eigenschaften aufweisen, denn diese für Bioelektroden günstigen elektrischen Eigenschaften kann lediglich der Leiter aufweisen, der mit dem elektrischen Kontaktmedium in Verbindung steht.

Dieser Leiter kann dabei grundsätzlich jede beliebige Geometrie aufweisen, in bevorzugten Ausführungsbeispielen der Erfindung kann der Leiter jedoch als rotationssymmetrische oder im Wesentlichen quaderförmige Leiterscheibe ausgebildet sein. Diese Leiterscheibe kann dabei zumindest teilweise über den verformten, erweiterten Bereich vorstehen.

Um bei einer Elektrode ein geringes Rauschen und eine Depolarisation im Falle einer Defibrillation zu erreichen, werden aktuell Redoxpaare verwendet. Diese können oxidiert bzw. reduziert werden und nehmen dabei mindestens ein Elektron auf oder geben mindestens ein Elektron ab. Aktuell werden verschiedenste Substanzen für diese Depolarisation verwendet. Am häufigsten werden Silber / Silberchlorid und Zinn / Zinnchlorid verwendet. Für die vorliegende Erfindung sind allerdings sämtliche Redoxpaare denkbar, welche eine Depolarisation der Elektrode ermöglichen. Dabei können die Redoxpaare aktiv beigemengt oder durch Reaktionen möglicherweise in situ erzeugt werden. Nachdem beispielsweise Silber / Silberchlorid eine relativ teure Substanz ist, reicht es aus, wenn gemäß einem weiteren Aspekt der Erfindung vorgesehen ist, dass der Leiter erfindungsgemäß einseitig mit einem elektrisch leitenden Material versehen ist, welches mit dem Anschlusselement und mit dem Kontaktmedium galvanisch verbunden ist.

Durch die Maßnahme, den Leiter erfindungsgemäß einseitig mit einem elektrisch leitenden Material zu versehen, kann man weiter Kosten sparen. Der eigentliche Leiter kann nämlich kostengünstige Materialien, wie beispielsweise Metall oder Kunststoff verwenden, während man an dem für die günstigen elektrischen Eigenschaften der Bioelektrode kritischen Übergangsbereich zum elektrischen Kontaktmedium (insbesondere Gel) ein zweites, elektrisch leitendes Material wie beispielsweise Silber / Silberchlorid verwenden kann. Es reicht aus, wenn dieses Material nur lokal in diesem Bereich vorhanden ist.

Insbesondere kann der Leiter aus einer mit einem elektrisch leitenden Material versehen Kunststofffolie bestehen.

Insgesamt liegt der Erfindung der Grundgedanke zu Grunde, das Anschlusselement für den Signalleiter so auszubilden, dass es gut in der Elektrode verankert ist, während es auf die elektrischen Eigenschaften weniger ankommt und damit kostengünstige Materialien verwendet werden können.

Andererseits können die für die günstige elektrische Signalleitung vorgesehenen teureren Materialen lediglich in dem elektrisch kritischen Bereich am Übergang zum elektrischen Kontaktmedium (Gel) verwendet werden. Diese Aufgabe übernimmt der Leiter. Ganz kurz formuliert würde man sagen, das elektrisch leitende Anschlusselement ist, abgesehen von der Grundeigenschaft der elektrischen Leitung hauptsächlich für die "Mechanik" verantwortlich. Beim Leiter ist es umgekehrt: er braucht keine besondere mechanischen Eigenschaften zu erfüllen und lediglich im Bereich der Übergangsstelle zum elektrischen Kontaktmedium (Gel) aus dafür günstigen Materialien bestehen. Insofern ist der Querleiter ohne besondere mechanische Aufgaben für die "Elektrik" zuständig.

Weitere Vorteile und Einzelheiten der Erfindung werden anhand der nachfolgenden Figurenbeschreibung näher erläutert. Darin zeigen:
- Fig. 1: eine schematische Unteransicht (später die der Haut zugewandte Seite) der Herstellungsschritte eines Ausführungsbeispiels einer erfindungsgemäßen Elektrode bis zur fertigen Elektrode,
- Fig. 2: eine schematische Draufsicht der Herstellungsschritte eines Ausführungsbeispiels einer erfindungsgemäßen Elektrode bis zur fertigen Elektrode, wobei nur ein Teil der Prozessschritte in einer Draufsicht dargestellt ist,
- Fig. 3: eine Abfolge der Schnitte gemäß der Linie A-A der Figur 1, wobei die Darstellung zur besseren Visualisierung als schematische Darstellung zu verstehen ist,
- Fig. 4: eine schematische Unteransicht (später die der Haut zugewandte Seite) der Herstellungsschritte eines weiteren Ausführungsbeispiels einer erfindungsgemäßen Elektrode bis zur fertigen Elektrode,
- Fig. 5: eine schematische Draufsicht der Herstellungsschritte eines weiteren Ausführungsbeispiels einer erfindungsgemäßen Elektrode bis zur fertigen Elektrode, wobei nur ein Teil der Prozessschritte in einer Draufsicht dargestellt ist,
- Fig. 6: eine Abfolge der Schnitte gemäß der Linie A-A der Figur 4, wobei die Darstellung zur besseren Visualisierung als schematische Darstellung zu verstehen ist,
- Fig. 7: eine schematische Unteransicht (später die der Haut zugewandte Seite) der Herstellungsschritte eines weiteren Ausführungsbeispiels einer erfindungsgemäßen Elektrode bis zur fertigen Elektrode,
- Fig. 8: eine schematische Draufsicht der Herstellungsschritte eines weiteren Ausführungsbeispiels einer erfindungsgemäßen Elektrode bis zur fertigen Elektrode, wobei nur ein Teil der Prozessschritte in einer Draufsicht dargestellt ist,
- Fig. 9: eine Abfolge der Schnitte gemäß der Linie A-A der Figur 7, wobei die Darstellung zur besseren Visualisierung als schematische Darstellung zu verstehen ist,
- Fig. 10: eine schematische Unteransicht eines Ausführungsbeispiels eines erfindungsgemäßen Trägers mit einer Klebstoffschicht,
- Fig. 11: eine schematische Unteransicht eines weiteren Ausführungsbeispiels eines erfindungsgemäßen Trägers mit einer Klebstoffschicht,
- Fig. 12a: eine schematische Seitenansicht eines Ausführungsbeispiels eines erfindungsgemäßen Anschlusselements,
- Fig. 12b: eine schematische Draufsicht eines Ausführungsbeispiels eines erfindungsgemäßen Anschlusselements,
- Fig. 13a: eine schematische Seitenansicht eines Verankerungsvorgangs eines erfindungsgemäßen Anschlusselements in einem Träger,
- Fig. 13b: eine schematische Draufsicht (später die der Haut abgewandte Seite) eines Verankerungsvorgangs eines erfindungsgemäßen Anschlusselements in einem Träger,
- Fig. 14a: eine schematische Ansicht eines weiteren Ausführungsbeispiels eines erfindungsgemäßen Anschlusselements,
- Fig. 14b: eine schematische Ansicht eines weiteren Ausführungsbeispiels eines erfindungsgemäßen Anschlusselements,
- Fig. 15a: eine schematische Seitenansicht eines weiteren Ausführungsbeispiels eines erfindungsgemäßen Anschlusselements,
- Fig. 15b: eine schematische Draufsicht eines weiteren Ausführungsbeispiels eines erfindungsgemäßen Anschlusselements,
- Fig. 16a: eine schematische Seitenansicht eines weiteren Ausführungsbeispiels eines erfindungsgemäßen Anschlusselements,
- Fig. 16b: eine schematische Draufsicht eines weiteren Ausführungsbeispiels eines erfindungsgemäßen Anschlusselements,
- Fig. 17a: eine schematische Seitenansicht eines weiteren Ausführungsbeispiels eines erfindungsgemäßen Anschlusselements, und
- Fig. 17b: eine schematische Draufsicht eines weiteren Ausführungsbeispiels eines erfindungsgemäßen Anschlusselements.
- Fig. 18: eine schematische Unteransicht (später die der Haut zugewandte Seite) der Herstellungsschritte eines weiteren Ausführungsbeispiels einer erfindungsgemäßen Elektrode, bis zur fertigen Elektrode.
- Fig. 19: eine schematische Draufsicht der Herstellungsschritte.
- Fig. 20: eine Abfolge gemäß den Figuren 18 und 19 in einer Schnittdarstellung.
- Fig. 21, 22 und 23: ähnliche Darstellungen wie in den Figuren 18,19 und 20, jedoch für ein weiteres Ausführungsbeispiel.

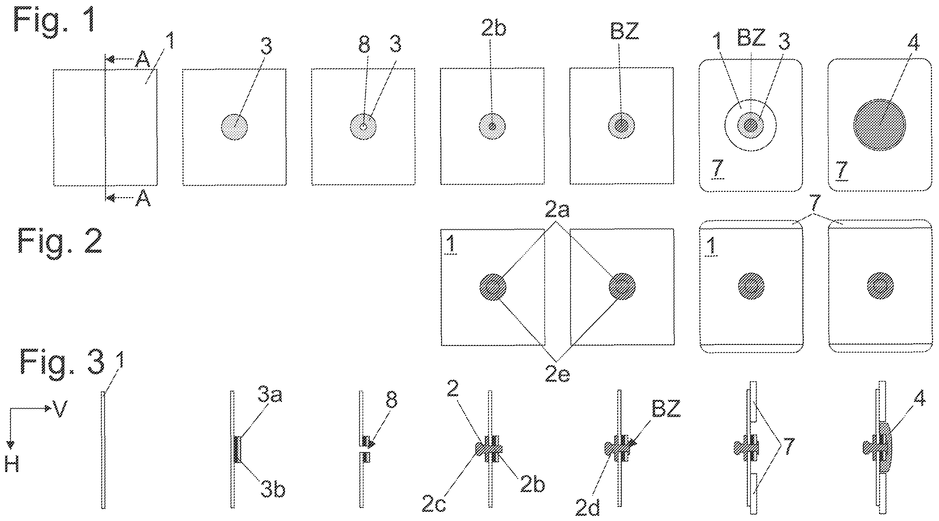

Unter Bezugnahme auf die Figuren 1 bis 3 wird nun der Verfahrensablauf zur Herstellung eines Ausführungsbeispiels einer erfindungsgemäßen Elektrode zum Anbringen auf der menschlichen Haut näher erläutert.

Ausgegangen wird von einem elektrisch nichtleitenden Träger 1. Das Trägermaterial dient zur Verankerung der elektrischen Komponenten der Elektrode. Es kann beispielsweise aus einer (flexiblen) Folie (beispielsweise aus PET oder TPU) bestehen, die auf der in der Zeichnung der Figur 1 nach oben weisenden Unterseite vollständig oder teilweise mit einem Klebstoff beschichtet ist, der beispielsweise selbstklebend (pressure sensitive adhesive) oder thermoaktivierbar (hot melt) ausgeführt sein kann.

Auf dieses Trägermaterial wird nun in einem nächsten Schritt ein rotationssymmetrischer Leiter 3 befestigt, insbesondere verklebt oder aufgedruckt. Der Leiter weist gemäß einer bevorzugten Variante der Erfindung zwei unterschiedlich elektrisch leitende Materialien oder ein elektrische nichtleitendes Material 3b und ein elektrisch leitendes Material 3a auf, wobei das elektrisch leitende Material 3a bzw. eines der beiden elektrisch leitenden Materialien später mit dem elektrischen Anschlusselement 2 und mit dem Kontaktmedium 4 (Gel) galvanisch verbunden wird.

Bei dem dargestellten Ausführungsbeispiel handelt es sich um einen schwarz bzw. grau dargestellten, kreisförmigen Leiter 3 aus einer Kunststofffolie. Der Leiter 3 kann aber auch aus einem Metall oder einem leitfähigen mit Kohlenstofffasern dotiertem Kunststoff bestehen.

Im Bereich der späteren Kontaktstelle mit dem elektrischen Kontaktmedium 4 (Gel) ist dieser Leiter 3 mit einer Schicht 3a aus beispielsweise Silber / Silberchlorid oder Zinn / Zinnchlorid oder einem anderen Redoxpaar beschichtet.

In einem weiteren Schritt wird nun eine Öffnung 8 durch den elektrischen Leiter 3 und den Träger 1 vorgesehen. Das kann beispielsweise durch Ausstanzen geschehen. Anschließend folgt die Einbringung des Anschlusselementes 2, das einen Vorsprung 2b aufweist, welcher über die Unterseite des Trägers 1 und des Leiters 3 vorsteht.

Das Anschlusselement 2 weist beim dargestellten Ausführungsbeispiel im Anschluss an den im Wesentlichen kugelförmigen Kopf 2c einen im Durchmesser reduzierten Hals 2d auf, an dem sich ein flanschförmig seitlich abstehender Haltebereich 2e und ein Vorsprung 2b anschließt

Insgesamt ist der seitlich abstehende flanschförmige Haltebereich 2e im Wesentlichen tellerförmig ausgebildet. Er dient zur Verteilung und Übertragung von auf das Anschlusselement 2 aufgebrachten Druckkräften auf den Träger 1 zuständig.

Kommt ein Anschlusselement 2 zum Einsatz, das aus einem einzigen Teil besteht, das einerseits mit dem elektrischen Leiter 3 in Verbindung steht und andererseits die Anschlussstelle 2a zum lösbaren Anschließen eines (hier nicht dargestellten) Signalleiters aufweist, ist damit eine kostengünstige Herstellung der Elektrode möglich, weil das meist kostenintensive Eyelet (Unterknopf) entfallen kann. Für die mechanische Verankerung reicht die einteilige Ausbildung des Anschlusselementes aus.

Die Anforderungen an die elektrischen Eigenschaften sind gering. Damit können einfache Konstruktionen, wie beispielsweise ein tiefgezogenes Metallteil als Anschlusselement 2 verwendet werden. Die etwas diffizileren elektrischen Aufgaben übernimmt also hier nicht das sonst übliche Eyelet (Unterknopf) sondern der Leiter 3, der mit dem später aufgebrachten elektrischen Kontaktmedium 4 (Gel) in Verbindung steht.

Es erfolgt also eine Trennung der Aufgaben. Das elektrische Anschlusselement 2 ist abgesehen von der Basiseigenschaft elektrisch leitend zu sein, im Wesentlichen für den mechanischen Halt in der Elektrode verantwortlich, während der Leiter 3 von mechanischen Aufgaben weitgehend befreit ist. Das erlaubt es eine günstige Materialwahl zu treffen. Insbesondere ist es möglich, teurere - aus elektrischer Sicht günstige - Materialien nur dort (Stelle 3a) vorzusehen, wo später der Kontakt mit dem Gel erfolgt.

Das elektrisch leitende Anschlusselement 2 kann - wie bereits erwähnt - aus einem tiefgezogenen Metallblech bestehen. Es ist dann im Inneren zumindest teilweise hohl. Es kann aber auch aus einem leitfähigen Kunststoff bestehen, beispielsweise aus ABS, das mit leitfähigen Karbonfasern dotiert ist.

Günstigerweise wird man das Anschlusselement im Wesentlichen rotationssymmetrisch ausbilden. Andere Varianten sind auch möglich.

Um das elektrische Anschlusselement 2 endgültig in der Elektrode zu fixieren und insbesondere auch gegen Zugbelastungen zu sichern, wird in einem nächsten Schritt der Vorsprungs 2b derart verformt, dass ein verformter, erweiterter Bereich BZ hergestellt wird.

Die Verformung des Vorsprungs 2b kann dabei durch Aufschmelzen, Umbördeln, Aufspreizen oder Umbiegen erfolgen. Es ist aber auch jedes andere geeignete Verfahren anwendbar.

Durch die Verformung des Vorsprungs 2b wird über den verformten, erweiterten Bereich BZ eine galvanische Verbindung zwischen dem Anschlusselement 2 und dem leitenden Material 3a des Leiters 3 und andererseits mittels Form- und/oder Kraftschluss eine mechanische Befestigung des Anschlusselements 2 an dem Träger 1 hergestellt.

Auf die Unterseite des Trägers 1 wird nun eine Pflasterschicht 7 aufgebracht, insbesondere verklebt, wobei die Pflasterschicht vorzugsweise mittels einer patientenseitigen Beschichtung aus biokompatiblem Klebstoff auf die Haut aufklebbar ist, um die Elektrode zu fixieren.

Dabei ist es auch möglich, die Pflasterschicht über eine auf ihr aufgebrachte Schicht aus Selbstkleber oder aus einem thermoaktivierbaren Kleber mit dem Träger 1 zu verkleben.

Das Pflastermaterial dient letztlich dazu, die Elektrode auf der Patientenhaut zu fixieren. Geeignete Pflastermaterialien können beispielsweise aus einer Folie (beispielsweise PE), aus einem Schaumband (beispielsweise PE-Schaum) oder aus Vliesstoffen bestehen. Die Pflastermaterialien sind üblicherweise patientenseitig mit einem biokompatiblen Klebstoff beschichten.

In einem letzten Herstellungsschritt der Elektrode gemäß den Figuren 1 bis 3 erfolgt die Einbringung des elektrischen Kontaktmediums 4 in eine dafür vorgesehene Aussparung im Pflastermaterial 7. Das elektrische Kontaktmedium 4 ermöglicht die (vorzugsweise ionen-basierte) Leitung von körpergenerierten elektrischen Potenzialen oder von gerätegenerierten Mess- oder Stimulationsströmen von der Körperoberfläche (Haut) zum elektrischen Anschlusselement 2 und umgekehrt. Das Kontaktmedium 4 kann beispielsweise aus einem mit Chloriden dotiertem Gel bestehen, das entweder in mehr oder weniger flüssiger Form (mehr oder weniger geliert) oder als quervernetzte Polymer-Matrix (Hydrogel) vorliegt. Es ist aber auch möglich, das elektrische Kontaktmedium 4 mit anderen Mitteln zu erzeugen, beispielsweise als leitfähiger Kleber oder als mit Salzlösung gefüllter Schwamm.

Jedenfalls wird das elektrische Kontaktmedium 4, wie es der letzte Schritt in den Figuren 1 bis 3 zeigt, in die Aussparung im Pflastermaterial 7 eingebracht. Es kontaktiert darin das elektrisch leitenden Material 3a (insbesondere Silber / Silberchlorid).

Das Zusammenwirken des elektrisch leitenden Materials 3a, insbesondere die Beschichtung mit Silber / Silberchlorid oder einem anderen geeigneten Material einerseits und dem Material des elektrisch leitenden Kontaktmediums 4 andererseits, erlaubt es günstige elektrische Eigenschaften der Elektrode, wie beispielsweise rauschfreie Signalübertragung oder depolarisierende Wirkungen zu erzielen, wobei der Einsatz des relativ teuren elektrisch leitenden Materials 3a des Leiters 3 auf jenen Bereich beschränkt bleiben kann, in den ein Kontakt mit dem Kontaktmedium 4 erfolgt. Das senkt die Kosten weiter.

Insgesamt ergibt sich bei der Herstellung gemäß den Figuren 1 bis 3 eine "zentrale" Elektrode, bei der das Anschlusselement 2 und das Kontaktmedium 4 (Gel) direkt übereinander angeordnet sind.

Die für das Ausführungsbeispiel gemäß den Figuren 1 bis 3 wesentlichen Verfahrensschritte sind die Folgenden:
- Anordnen, vorzugsweise Verkleben oder Aufdrucken, eines Leiters (3) auf der der Haut zugewandten Unterseite eines elektrisch nichtleitenden Trägers (1),
- Einbringen eines Anschlusselementes (2) von der Oberseite des Träger durch diesen hindurch, derart dass auf der gegenüberliegenden Unterseite des Trägers (1) der Vorsprung (2b) des Anschlusselements (2) vorsteht und das Anschlusselement (2) - vorzugsweise mit einem seitlich abstehenden, tellerförmigen Haltebereich (2e) - an der Oberseite des Trägers (1) anliegt,
- Verformen des Vorsprungs (2b) des Anschlusselements (2) derart, dass ein verformter, erweiterter Bereich (BZ) hergestellt wird, welcher einerseits eine elektrisch leitfähige Verbindung zwischen dem Anschlusselement (2) und dem Leiter (3) und andererseits eine mechanische Befestigung des Anschlusselements (2) am Träger (1) hergestellt.

Schließlich werden dann zur Fertigstellung der Elektrode noch folgende Schritte gesetzt:
- Anbringen - vorzugsweise Verkleben - einer hautseitig klebenden Pflasterschicht 7 mit dem Träger 1,
- Einbringen von einem elektrischen Kontaktmedium 4 - vorzugsweise einem Gel - in eine Aussparung der Pflasterschicht 7, derart dass der darunterliegende Leiter 3 kontaktiert wird.

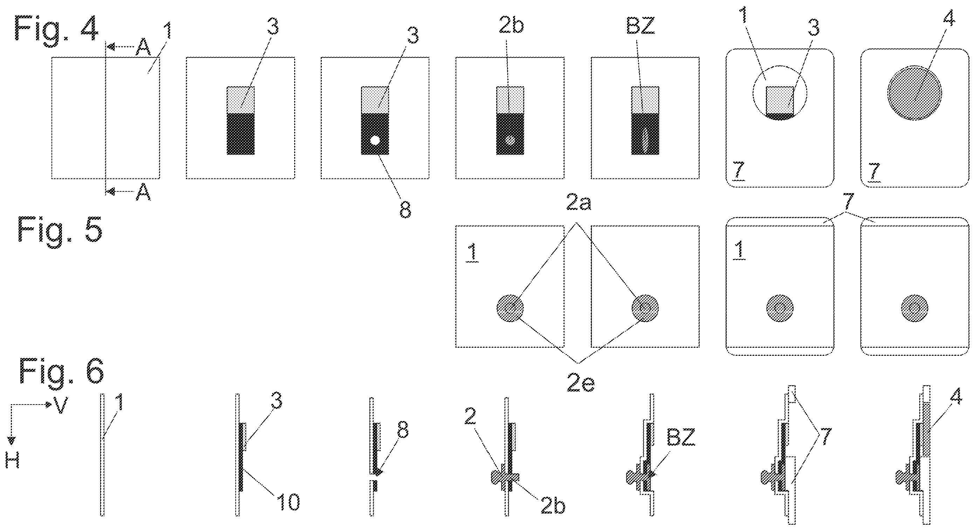

Bei dem in den Figuren 4 bis 6 dargestellten Ausführungsbeispiel stimmen die meisten Verfahrensschritte mit denen in Figur 1 bis 3 überein, weshalb auch gleiche Bezugszeichen gleiche Teile bezeichnen.

Der Unterschied besteht im Wesentlichen darin, dass eine "dezentrale" Elektrode vorgesehen wird. Das heißt, das Kontaktmedium 4 einerseits und das Anschlusselement 2 andererseits sind in einer horizontalen Ebene H gegeneinander versetzt.

Bei einem solchen Ausführungsbeispiel ist es notwendig, einen elektrisch leitenden Querleiter 10 vorzusehen, welcher eine galvanische Verbindung zwischen dem Leiter 3 und dem Anschlusselement 2 herstellt.

Bei der Verformung des Vorsprungs 2b kann Druck auf die Schichten ausgeübt werden, sodass sich diese entsprechend konturieren und miteinander verbinden. Der in Figur 6 gezeigte Querschnitt nach Anbringen der Pflasterschicht 7 mit den dort gezeigten Kanten ist allerdings lediglich als schematische Darstellung zu sehen. In Wirklichkeit sind die Schichtdicken meist geringer und die Verläufe der Schichten sind wesentlich abgerundeter.

Es ist weiters ersichtlich, dass der verformte, erweiterte Bereich BZ nicht mehr kreisförmig, sondern lamellenartig ausgebildet ist. Der verformte, erweiterte Bereich BZ kann grundsätzlich aber jede beliebige Form aufweisen.

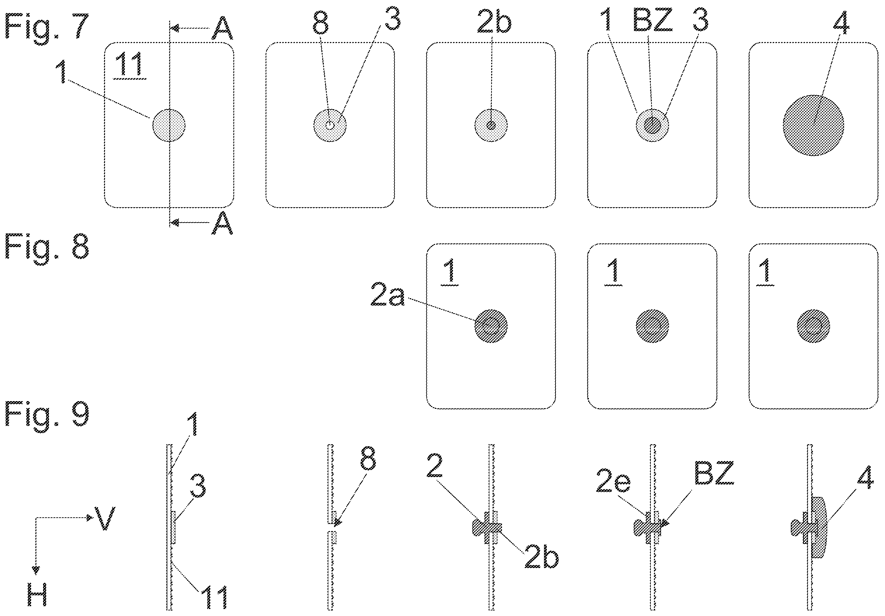

Bei dem in den Figuren 7 bis 9 dargestellten Ausführungsbeispiel stimmen die meisten Verfahrensschritte mit denen in Figur 1 bis 3 überein, weshalb auch gleiche Bezugszeichen gleiche Teile bezeichnen.

Der Unterschied besteht im Wesentlichen darin, dass auf dem Träger 1 eine biokompatible Klebstoffschicht 11 zum Aufkleben der Elektrode auf eine Haut eines Patienten vorgesehen ist. Somit kann die Pflasterschicht 7 entfallen und ein weiterer Prozessschritt eingespart werden.

Die Klebstoffschicht 11 kann dabei vor oder nach dem Aufbringen des Leiters 3 auf den Träger 1 aufgebracht werden bzw. ist die Klebstoffschicht 11 bereits am Ausgangsmaterial des Trägers 1 vorhanden.

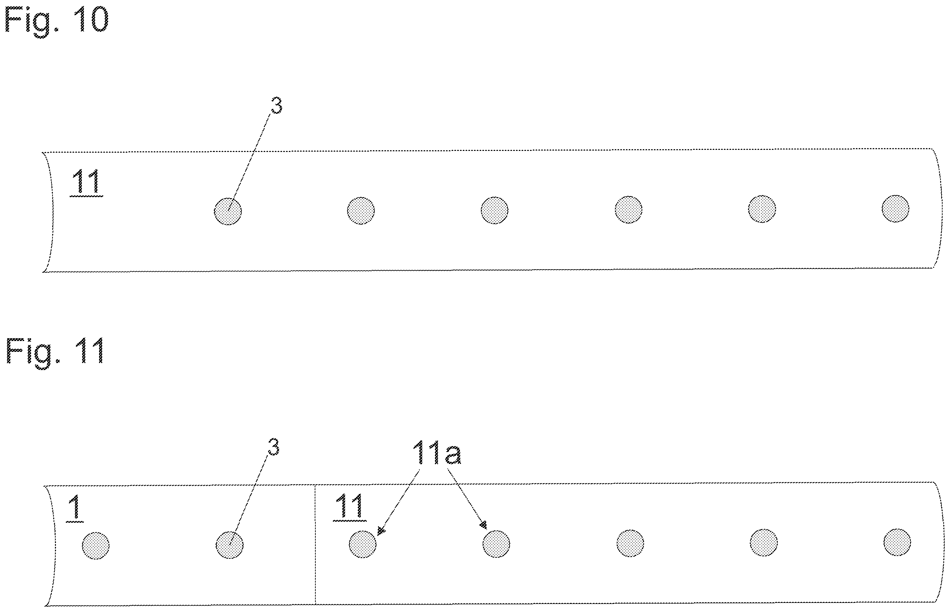

In den Figuren 10 und 11 sind die oben genannten Varianten zum Aufbringen des Klebstoffs 11 gezeigt.

In Figur 10 ist der Klebstoff 11 bereits am Träger 1 vorhanden bzw. wird vor dem Aufbringen des Leiters 3 aufgebracht. Der Leiter 3 wird dabei anschließend auf die Klebstoffschicht 11 aufgebracht. Dabei kann der Leiter 3 durch die Klebstoffschicht 11 gehalten werden, wodurch der Leiter 3 nicht zusätzlich mit dem Träger 1verklebt werden muss.

In Figur 11 wird der Leiter 3 auf den Träger 1 aufgebracht und anschließend der Klebstoff 11 auf den Träger 1 aufgebracht. Dabei ist eine Ausnehmung 11a vorgesehen, damit der Leiter 3 nicht von dem Klebstoff 11 verdeckt wird.

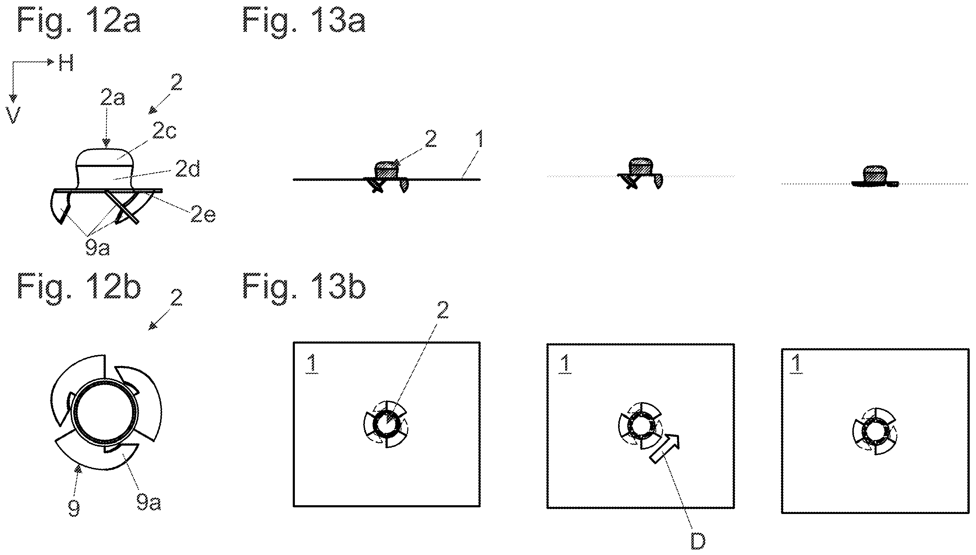

Die Figuren 12a und 12b zeigen ein Ausführungsbeispiel eines erfindungsgemäßen Anschlusselements 2. Es ist erkennbar, dass das Anschlusselement 2 Flügelsegmente 9 aufweist, welche sowohl den Vorsprung als auch den Haltebereich des Anschlusselements 2 bilden. Die gegenüber einer horizontalen Lage H geneigten Flügelsegmentabschnitte 9a können dabei gleich oder unterschiedlich lang ausgebildet sein. Es ist auch denkbar, dass die Flügelsegmente 9 zumindest abschnittsweise scharfkantig ausgebildet sind, um ein Durchdringen eines Trägers 1 und eines Leiters 3 zu erleichtern.

Die Figuren 13a und 13b zeigen schematische Ansichten eines Verankerungsvorgangs eines erfindungsgemäßen Anschlusselements in einem Träger, mit einem wie in den Figuren 12a und 12b dargestellten Anschlusselement 2.

In einem ersten Schritt wird dazu das Anschlusselement 2 von einer später der Haut abgewandten Oberseite eines Trägers 1 durch den Träger 1 und den auf der Unterseite des Trägers 1 angebrachten, nicht dargestellten Leiter 3 gedrückt. Das heißt, das Anschlusselement 2 durchdringt den Träger 1 und den Leiter 3 mit den Flügelsegmentabschnitten 9a.

In einem nächsten Schritt wird das Anschlusselement 2 in eine Richtung D verdreht. Dadurch erfolgt eine bessere Verankerung des Anschlusselements 2 im Träger 1.

In einem letzten Schritt werden die Flügelsegmentabschnitte 9a in Richtung der Unterseite des Trägers 1 über eine horizontale Lage H hinaus aufgebogen, wodurch eine Klemmung des Trägers 1 und des Leiters 3 erreicht wird. Somit ist auch eine elektrische Verbindung des Anschlusselements 2 mit dem Leiter 3 und eine mechanische Befestigung des Anschlusselements 2 am Träger 1 sichergestellt. Es ist allerdings auch möglich, dass die Flügelsegmentabschnitte nur aufgebogen werden, bis sie sich in einer horizontale Lage H befinden.

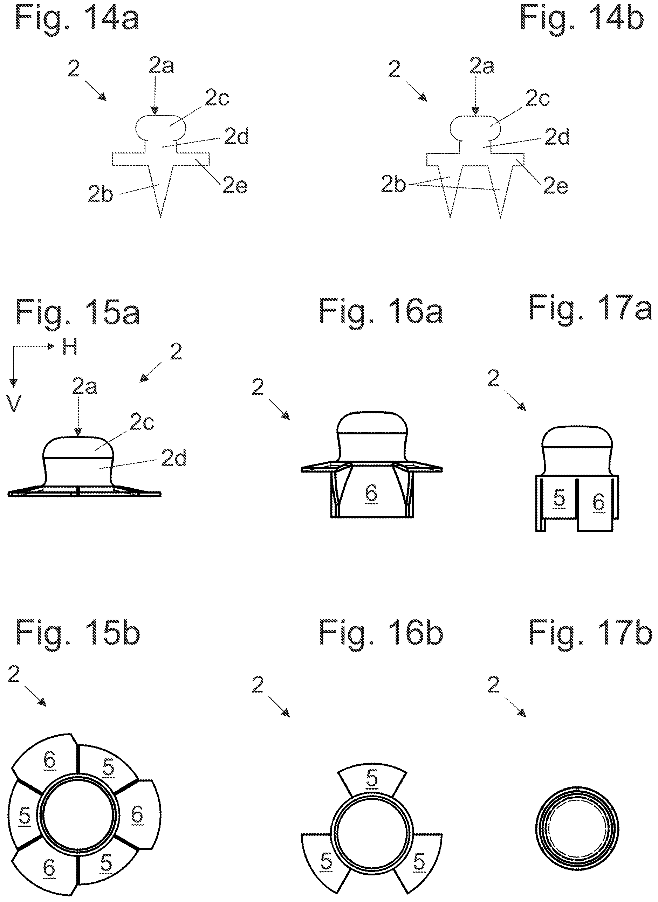

Die Figur 14a zeigt ein Ausführungsbeispiel eines Anschlusselements 2 bei welchem der Vorsprung 2b als sich verjüngender Dorn ausgebildet ist. Dadurch ist es möglich, das Anschlusselement 2 ohne vorherigem Herstellen einer durchgehenden Öffnung 8 durch den Leiter 3 und den Träger 1 in den Träger 1 bzw. Leiter 3 einzubringen. Es wird also ein Arbeitsschritt eingespart.

Figur 14b zeigt ein Ausführungsbeispiel eines Anschlusselements 2, bei welchem zwei Vorsprünge 2b als sich verjüngende Dorne ausgebildet sind. Es kann allerdings eine beliebige Anzahl an Vorsprüngen 2b vorgesehen sein. Außerdem können auch mehrere Vorsprünge 2b, welche nicht dornförmig ausgebildet sind, vorgesehen sein. Die Mehrzahl an Vorsprüngen 2b kann dabei rotationssymmetrisch oder nicht rotationssymmetrisch am Anschlusselement 2 angeordnet sein.

Die Figuren 15a bis 17b zeigen Ausführungsbeispiele eines Anschlusselements 2, bei welchem der Vorsprung und der flanschartige Haltebereich des Anschlusselements 2 aus zumindest einem ersten Segment 5 und zumindest einem zweiten Segment 6 gebildet werden.

Es ist auch ersichtlich, dass die zweiten Segmente 6 länger als die ersten Segmente 5 ausgebildet sind. Die Segmente 5, 6 können auch gleich lang ausgebildet sein, oder die Segmente 5 können länger als die Segmente 6 ausgebildet sein.

Figur 15a zeigt das Anschlusselement in einer Frontalansicht, wenn sich alle Segmente 5, 6 in einer Horizontalen Lage H befinden. Figur 15b zeigt die entsprechende Draufsicht.

Figur 16a zeigt das Anschlusselement in einer Frontalansicht, wenn sich die Segmente 5 in einer horizontalen Lage H und die Segmente 6 sich in einer vertikalen Lage V befinden. Figur 16b zeigt die entsprechende Draufsicht.

Figur 17a zeigt das Anschlusselement in einer Frontalansicht, wenn sich alle Segmente 5, 6 in einer vertikalen Lage H befinden. Figur 17b zeigt die entsprechende Draufsicht.

Bei einem Anschlusselement 2 gemäß den Figuren 15a und 15b wird vor dem Einbringen des Anschlusselements 2 in den Träger 1 zumindest ein erstes Segment 5 der zumindest zwei Segmente 5,6 in eine vertikale Lage V überführt und nach dem Einbringen des Anschlusselements 2 in den Träger 1 das zumindest eine erste Segment 5 wieder in eine horizontale Lage H überführt.

Bei einem Ausführungsbeispiel eines Anschlusselements 2 gemäß den Figuren 17a und 17b wird vor dem Einbringen des Anschlusselements 2 in den Träger 1 zumindest ein erstes Segment 5 in eine horizontale Lage H überführt und nach dem Einbringen des Anschlusselements 2 in den Träger 1 zumindest ein zweites Segment 6 in eine horizontale Lage H überführt wird.

Bei den bisherigen Ausführungsbeispielen gemäß den Figuren 1 bis 17b waren der Leiter 3 und das Kontaktmedium 4 von gesonderten Bauteilen gebildet und bestehen auch aus vorzugsweise unterschiedlichen Materialien.

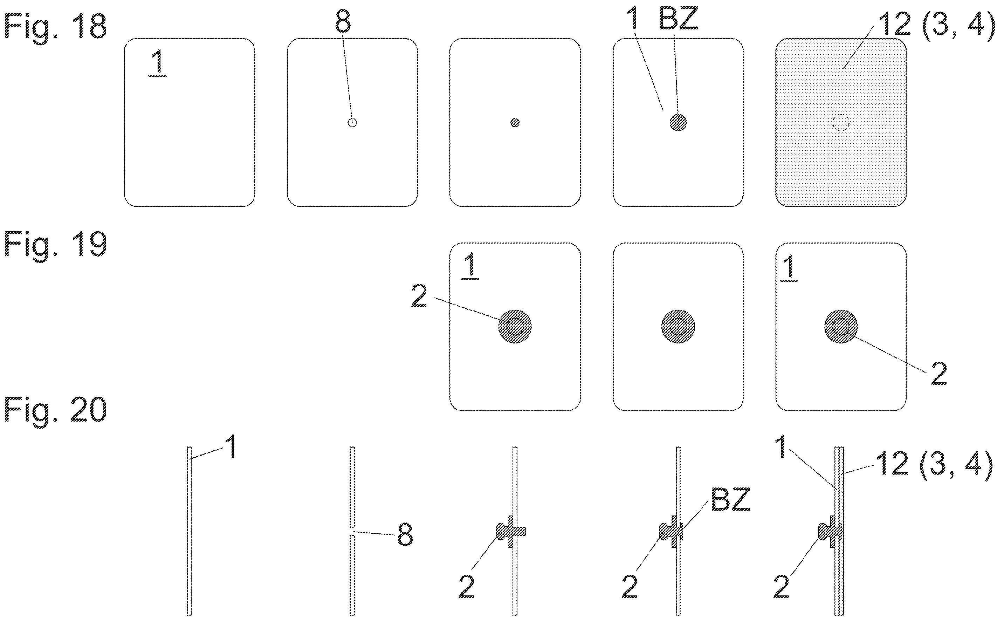

Es besteht aber auch die Möglichkeit, dass der Leiter 3 und das Kontaktmedium 4 von ein und demselben Bauteil, vorzugsweise von einem elektrisch leitfähigen Kleber gebildet wird, womit man sich insgesamt Bauteile einsparen kann. Das wird im Folgenden anhand der Figuren 18 bis 23 näher erläutert.

Bei dem in den Figuren 18 und 20 dargestellten Ausführungsbeispiel wird zunächst in einem Träger eine Öffnung 18 angebracht, in diese wird ein Anschlusselement eingeschoben und anschließend der durchstehende Bereich durch Verformen erweitert. Der erweiterte Bereich BZ hält dann das Anschlusselement 2 sicher am Träger. Anschließend wird eine Schicht aus elektrisch leitfähigem Kleber 12 aufgebracht, mittels dem die fertige Elektrode auf der Haut des Patienten aufgeklebt werden kann. Die elektrisch leitfähige Schicht aus dem Kleber 12 übernimmt daher die Funktion des Leiters 3 und des Kontaktmediums 4.

Bei der in den Figuren 18 und 20 dargestellten Ausführungsform ist der Kleber 12 auf der Unterseite des Trägers 1 vollflächig aufgebracht.

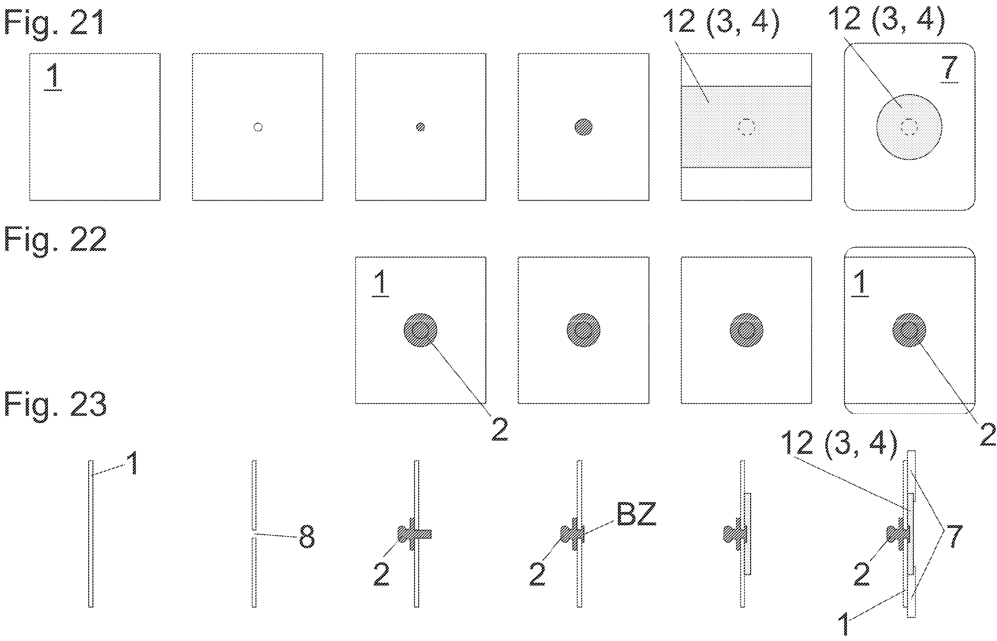

Bei dem in den Figuren 21 bis 23 dargestellten Ausführungsbeispiel sind die meisten Herstellungsschritte identisch wie bei dem Beispiel gemäß den Figuren 18 bis 20. Lediglich im vorletzten Schritt wird der elektrisch leitfähige Kleber 12, der die Funktion des Leiters 3 und des Kontaktmediums 4 übernimmt, nicht vollflächig, sondern nur partiell - wie die Figur 21 zeigt, in Form eines Streifens - aufgebracht.

Zusätzlich wird in einem letzten Schritt ein ebenfalls klebendes Pflastermaterial 7 mit einer zentralen Öffnung aufgebracht, durch die der elektrisch leitfähige Kleber als Kontaktmedium noch frei liegt. Insgesamt erfolgt also die Verklebung mit der Haut einerseits über das klebende Pflastermaterial 7 und andererseits über den elektrisch leitfähigen Kleber 12. Der Vorteil der Variante gemäß den Figuren 21 bis 23 ist eine exzellente Verklebung durch das klebende Pflastermaterial 7 und eine Einsparung an elektrisch leitfähigem Kleber gegenüber der vollflächigen Variante gemäß den Figuren 18 bis 20. Dafür kann bei den letztgenannten Figuren auf ein zusätzliches Bauelement, nämlich das Pflastermaterial 7, verzichtet werden.

Bei den Ausführungsbeispielen der Erfindung kann insbesondere eine zweite Schicht des Leiters - wie oben beschrieben - durch eine Schicht Silber/Silberchlorid oder Zinn/Zinnchlorid oder ein anderes Redoxpaar gebildet sein.

Es ist aber auch möglich, dass darüber hinaus andere elektrisch leitende Komponenten mit solchen Redoxpaaren versehen sind, insbesondere auch das Kontaktmedium 4 und/oder das Anschlusselement 2. Im Sinne eines sparsamen Umganges mit den relativ teuren Redoxkomponenten wird man nicht alle leitfähigen Elemente gleichzeitig mit solchen Redoxpaaren versehen, wenngleich das auch theoretisch möglich ist. Es reicht - wie bereits erwähnt - aus, wenn eine zweite Schicht des Leiters 3 mit einem solchen Redoxpaar versehen ist. Grundsätzlich ist es auch möglich, die Redoxpaare überhaupt einzusparen und vorzusehen, dass weder das Anschlusselement 2 noch der Leiter 3, noch das Kontaktmedium 4 ein solches Redoxpaar enthalten.

### Bezugszeichenliste:

- 1.: Träger
- 2.: Anschlusselement
- 2a.: Anschlussstelle
- 2b.: Vorsprung
- 2c.: Kopf
- 2d.: Hals
- 2e.: Haltebereich
- 3.: Leiter
- 3a.: elektrisch leitendes Material
- 3b.: elektrisch nichtleitendes Material
- 4.: Kontaktmedium
- 5.: Erstes Segment
- 6.: Zweites Segment
- 7.: Pflasterschicht
- 8.: Öffnung
- 9.: Flügelsegment
- 9a.: Flügelsegmentabschnitt
- 10.: Querleiter
- 11.: Klebeschicht (Hautkleber)
- 11a.: Ausnehmung
- 12: elektrisch leitfähiger Kleber

- H: Horizontale
- V: Vertikale
- BZ: Verformter, erweiterter Bereich

## Patentansprüche

1. Elektrode zum Anbringen auf der menschlichen Haut mit einem elektrisch nichtleitenden Träger (1), der auf seiner der Haut abgewandten Oberseite ein vorstehendes, elektrisch leitendes Anschlusselement (2) mit einer Anschlussstelle (2a) zum lösbaren Anschließen eines Signalleiters aufweist, wobei ein zumindest teilweise auf der gegenüberliegenden Unterseite des Trägers (1) angeordneter Leiter (3) vorgesehen ist, welcher elektrisch mit dem Anschlusselement (2) sowie mit einem der Haut zugewandten Kontaktmedium (4) in Verbindung steht, wobei das Anschlusselement (2) zumindest einen durch den Träger (1) reichenden Vorsprung (2b) aufweist, der an seinem Ende einen durch Verformung gebildeten, erweiterten Bereich (BZ) aufweist, wobei durch diesen verformten, erweiterten Bereich (BZ) einerseits eine elektrische Verbindung zwischen dem Leiter (3) und dem Anschlusselement (2a) und andererseits eine mechanische Befestigung des Anschlusselements (2) am Träger (1) herstellbar sind, wobei das Anschlusselement (2) einen im Wesentlichen kugelförmigen Kopf (2c), einen daran anschließenden, im Durchmesser reduzierten Hals (2d) und einen an das Ende des Halses (2d) anschließenden, flanschförmig seitlich abstehenden Haltebereich (2e) aufweist und wobei der zumindest eine Vorsprung (2b) an den Haltebereich (2e) anschließt, wobei der Leiter (3) einseitig mit einem elektrisch leitenden Material beschichtet ist und, wobei das elektrisch leitende Material von einem Paar Silber/Silberchlorid, Zinn/Zinnchlorid oder einem anderen beispielsweise zur Depolarisation einer Elektrode geeigneten Redoxpaar gebildet ist oder derartige Redoxpaare enthält und wobei das Anschlusselement (2) auf der Unterseite und der Oberseite des Trägers (1) mit diesem unter Zwischenschaltung des flächig ausgebildeten Leiters verbunden ist.

2. Elektrode nach Anspruch 1, **dadurch gekennzeichnet, dass** das Anschlusselement (2) aus einem einzigen Teil besteht, das die Anschlussstelle (2a) zum lösbaren Anschließen einer Signalleitung aufweist.

3. Elektrode nach einem der Ansprüche 1 oder 2, **dadurch gekennzeichnet, dass** der zumindest eine Vorsprung (2b) aus zumindest einem ersten Segment (5) und zumindest einem zweiten Segment (6) gebildet wird, wobei sich die zumindest zwei Segmente (5, 6) in einer Ausgangslage vorzugsweise in einer horizontalen Lage (H) oder in einer vertikalen Lage (V) befinden, oder wobei sich zumindest eines der der zumindest zwei Segmente (5, 6) in einer horizontalen Lage (H) und zumindest ein zweites der zumindest zwei Segemente (5, 6) in einer vertikalen Lage (V) befindet, und dass der flanschartige Haltebereich (2e) durch zumindest eines der zumindest zwei Segmente (5, 6) gebildet wird.

4. Elektrode nach einem der Ansprüche 1 bis 3, **dadurch gekennzeichnet, dass** der zumindest eine Vorsprung (2b) als ein sich in eine dem Haltebereich (2e) entgegengesetzte Richtung verjüngender Dorn ausgebildet ist.

5. Elektrode nach einem der Ansprüche 1 bis 4, **dadurch gekennzeichnet, dass** das Anschlusselement (2) zumindest zwei Flügelsegmenten (9) aufweist, wobei die zumindest zwei Flügelsegmente (9) einen gegenüber einer horizontalen Lage (H) geneigten Abschnitt (9a) aufweisen, wobei die Flügelsegmente den Vorsprung (2b) und den seitlich abstehende flanschartige Haltebereich (2e) bilden, wobei vorzugsweise die zumindest zwei Flügelsegmente (9) zumindest abschnittsweise scharfkantig ausgebildet sind.

6. Elektrode nach einem der Ansprüche 1 bis 5, **dadurch gekennzeichnet, dass** der Leiter (3) als zumindest teilweise über den verformten, erweiterten Bereich (BZ) vorstehende Leitscheibe ausgebildet ist.

7. Elektrode nach einem der Ansprüche 1 bis 6, **dadurch gekennzeichnet, dass** das Anschlusselement (2) einerseits und das Kontaktmedium (4) andererseits an seitlich gegeneinander versetzten Stellen am Träger (1) angeordnet sind.

8. Elektrode nach einem der Ansprüche 1 bis 7, **dadurch gekennzeichnet, dass** der Träger (1) auf der der Haut zugewandten Seite mit Klebstoff, vorzugsweise einem Hautkleber, beschichtet ist, der vorzugsweise selbstklebend oder thermoaktivierbar ausgeführt ist, oder eine mit einem Klebstoff, vorzugsweise einem Hautkleber, versehene Pflasterschicht (7) aufweist.

9. Elektrode nach einem der Ansprüche 1 bis 8, **dadurch gekennzeichnet, dass** der Leiter (3) und das Kontaktmedium (4) von gesonderten Bauteilen gebildet sind, und vorzugsweise aus unterschiedlichen Materialien bestehen (Fig. 1 bis 17b), oder dass
der Leiter (3) und das Kontaktmedium (4) von ein und demselben Bauteil - vorzugsweise von einem elektrisch leitfähigen Kleber, mit dem die Elektrode auf die Haut eines Patienten klebbar ist - gebildet sind (Fig. 18 bis 23).

10. Elektrode nach einem der Ansprüche 1 bis 9, **dadurch gekennzeichnet, dass** das Anschlusselement (2) und/oder das Kontaktmedium (4) zumindest abschnittsweise von einem Paar Silber/Silberchlorid, Zinn/Zinnchlorid oder einem anderen beispielsweise zur Depolarisation einer Elektrode geeigneten Redoxpaar gebildet ist oder derartige Redoxpaare enthält.

11. Verfahren zum Herstellen einer Elektrode zum Anbringen auf der menschlichen Haut nach einem der Ansprüche 1 bis 10, **gekennzeichnet durch**:
- Anordnen, vorzugsweise Verkleben oder Aufdrucken, eines Leiters (3) auf der der Haut zugewandten Unterseite eines elektrisch nichtleitenden Trägers (1), wobei der Leiter (3) einseitig mit einem elektrisch leitenden Material beschichtet wird und, wobei das elektrisch leitende Material von einem Paar Silber/ Silberchlorid, Zinn/Zinnchlorid oder einem anderen beispielweise zur Depolarisation einer Elektrode geeigneten Redoxpaar gebildet wird oder derartige Redoxpaare enthält,
- Einbringen eines Anschlusselementes (2) von der Oberseite des Trägers (1) durch diesen hindurch, derart dass auf der gegenüberliegenden Unterseite des Trägers (1) der Vorsprung (2b) des Anschlusselements (2) vorsteht und das Anschlusselement (2) - vorzugsweise mit einem seitlich abstehenden, tellerförmigen Haltebereich (2e) - an der Oberseite des Trägers (1) anliegt, wobei das Anschlusselement (2) auf der Unterseite und der Oberseite des Trägers (1) mit diesem unter Zwischenschaltung des flächig ausgebilldeten Leiters verbunden wird,
- Verformen des Vorsprungs (2b) des Anschlusselements (2) derart, dass ein verformter, erweiterter Bereichs (BZ) hergestellt wird, welcher einerseits eine elektrisch leitfähige Verbindung zwischen dem Anschlusselement (2) und dem Leiter (3) und andererseits eine mechanische Befestigung des Anschlusselements (2) am Träger (1) herstellt.

12. Verfahren nach Anspruch 11, **dadurch gekennzeichnet, dass** die Verformung des Vorsprungs (2b) erfolgt durch:
- Aufschmelzen des Vorsprungs (2b), und/oder
- Umbördeln des Vorsprungs (2b), und/oder
- Aufspreizen des Vorsprungs (2b), und/oder
- Umbiegen des Vorsprungs (2b).

## Claims

1. An electrode for application to the human skin having an electrically non-conducting carrier (1) which on its top side that faces away from the skin has a projecting electrically conducting connecting element (2) having a connecting location (2a) for releasable connection of a signal conductor, wherein there is provided a conductor (3) which is arranged at least partially on the opposite underside of the carrier (1) and which is electrically connected to the connecting element (2) and to a contact medium (4) that faces towards the skin, wherein the connecting element (2) has at least one projection (2b) which passes through the carrier (1) and which at its end has an enlarged region (BZ) formed by deformation, wherein on the one hand an electrical connection between the conductor (3) and the connecting element (2a) and on the other hand mechanical fixing of the connecting element (2) to the carrier (1) can be made by that deformed enlarged region (BZ), wherein the connecting element (2) has a substantially ball-shaped head (2c), a neck (2d) of reduced diameter adjoining the same and a holding region (2e) which adjoins the end of the neck (2d) and which projects laterally in a flange shape and wherein the at least one projection (2b) adjoins the holding region (2e), wherein the conductor (3) is coated with an electrically conducting material on one side, and wherein the electrically conducting material is formed by a pair silver/silver chloride or tin/tin chloride or another redox couple suitable for example for depolarization of the electrode or comprises such redox couples, and wherein the connecting element (2) is connected on the underside and the top side of the carrier (1) to the same with interposition of a flat conductor.

2. Electrode according to claim 1, **characterized in that** the connecting element (2) comprises a single part which has the connecting location (2a) for releasable connection of a signal line.

3. Electrode according to one of claims 1 or 2, **characterized in that** the at least one projection (2b) is formed from at least one first segment (5) and at least one second segment (6), wherein the at least two segments (5, 6) in a starting position are preferably in a horizontal position (H) or in a vertical position (V), or wherein at least one of the at least two segments (5, 6) is in a horizontal position (H) and at least a second of the at least two segments (5, 6) is in a vertical position (V) and that the flange-like holding region (2e) is formed by at least one of the at least two segments (5, 6).

4. Electrode according to one of claims 1 to 3, **characterized in that** the at least one projection (2b) is in the form of a spike narrowing in the direction opposite to the holding region (2e).

5. Electrode according to one of claims 1 to 4, **characterized in that** the connecting element (2) has at least two wing segments (9), wherein the at least two wing segments (9) have a portion (9a) inclined with respect to a horizontal position (H), wherein the wing segments form the projection (2b) and the laterally projecting flange-like holding region (2e), wherein preferably the at least two wing segments (9) are at least portion-wise of a sharp-edged configuration.

6. Electrode according to one of claims 1 to 5, **characterized in that** the conductor (3) is in the form of a conducting plate projecting at least partially beyond the deformed enlarged region (BZ).

7. Electrode according to one of claims 1 to 6, **characterized in that** the connecting element (2) on the one hand and the contact medium (4) on the other hand are arranged at laterally mutually displaced positions on the carrier (1).

8. Electrode according to one of claims 1 to 7, **characterized in that** the carrier (1) is coated on the side towards the skin with adhesive, preferably a skin adhesive, which is preferably a pressure sensitive adhesive or is thermo-activatable, or has a plaster layer (7) provided with an adhesive, preferably a skin adhesive.

9. Electrode according to one of claims 1 to 8, **characterized in that** the conductor (3) and the contact medium (4) are formed by separate components and preferably comprise different materials (Figures 1 through 17b), or that the conductor (3) and the contact medium (4) are formed by one and the same component - preferably by an electrically conductive adhesive with which the electrode can be glued to the surface of a patient (Figures 18 through 23).

10. Electrode according to one of claims 1 to 9, **characterized in that** the connecting element (2) and/or the contact medium (4) is at least portion-wise formed by a pair silver/silver chloride, tin/tin chloride or another redox couple suitable for example for depolarization of an electrode or such redox couples or comprises such redox couples.

11. Method for producing an electrode for application to the human skin according to one of claims 1 to 10, **characterized by**:
- arranging, preferably gluing or printing, a conductor (3) on the underside, towards the skin, of an electrically non-conducting carrier (1), wherein the conductor (3) is coated with an electrically conducting material on one side, and wherein the electrically conducting material is formed by a pair silver/silver chloride or tin/tin chloride or another redox couple suitable for example for depolarization of the electrode or comprises such redox couples,
- introducing a connecting element (2) from the top side of the carrier (1) through the same in such a way that the projection (2b) of the connecting element (2) projects on the opposite underside of the carrier (1) and the connecting element (2) bears against the top side of the carrier (1) - preferably with a laterally projecting plate-shaped holding region (2e), wherein the connecting element (2) is connected on the underside and the top side of the carrier (1) to the same with interposition of a flat conductor,
- deforming the projection (2b) of the connecting element (2) in such a way that there is produced a deformed enlarged region (BZ) which on the one hand makes an electrically conductive connection between the connecting element (2) and the conductor (3) and on the other hand provides for mechanical fixing of the connecting element (2) to the carrier (1).

12. Method according to claim 11, **characterized in that** deformation of the projection (2b) is effected by:
- fusing the projection (2b), and/or
- beading over the projection (2b), and/or
- spreading open the projection (2b), and/or
- bending over the projection (2b).

## Revendications

1. Électrode destinée à être appliquée sur la peau humaine, comprenant un support (1) électriquement non conducteur qui présente, sur sa face supérieure opposé à la peau, un élément de raccordement (2) saillant et électriquement conducteur, muni d'un point de raccordement (2a) pour le raccordement détachable d'un conducteur de signal, un conducteur (3) étant prévu, lequel est disposé au moins partiellement sur la face inférieure opposée du support (1), et est électriquement relié à l'élément de raccordement (2) ainsi qu'à un milieu de contact (4) orienté vers la peau, l'élément de raccordement (2) présentant au moins une saillie (2b) traversant le support (1), laquelle comporte à son extrémité une zone élargie (BZ) obtenue par déformation, ladite zone élargie (BZ) permettant d'une part d'établir une connexion électrique entre le conducteur (3) et l'élément de raccordement (2) et d'autre part d'assurer une fixation mécanique de l'élément de raccordement (2) sur le support (1), l'élément de raccordement (2) présentant une tête sensiblement sphérique (2c), un col (2d) de diamètre réduit qui y fait suite, et une zone de retenue (2e) s'écartant latéralement en forme de bride dans le prolongement du col (2d), ladite au moins une saillie (2b) étant raccordée à la zone de retenue (2e), le conducteur (3) étant revêtu d'un côté d'un matériau électriquement conducteur, lequel matériau étant constitué d'un couple argent/chlorure d'argent, étain/chlorure d'étain ou d'un autre couple rédox approprié par exemple pour la dépolarisation d'une électrode, ou contenant un tel couple, et l'élément de raccordement (2) étant relié au support (1) sur sa face inférieure et sur sa face supérieure avec interposition du conducteur de forme plane.

2. Électrode selon la revendication 1, **caractérisée en ce que** l'élément de raccordement (2) est constitué d'une seule pièce comportant le point de raccordement (2a) pour le raccordement détachable d'une ligne de signal.

3. Électrode selon l'une des revendications 1 ou 2, **caractérisée en ce que** ladite au moins une saillie (2b) est formée d'au moins un premier segment (5) et d'au moins un second segment (6), lesdits au moins deux segments (5, 6) dans une position initiale se trouvant de préférence dans une position horizontale (H) ou une position verticale (V), ou bien au moins un desdits au moins deux segments (5, 6) étant en position horizontale (H) et au moins un deuxième desdits au moins deux segments (5, 6) étant en position verticale (V), et **en ce que** la zone de retenue en forme de bride (2e) est formée par au moins un desdits au moins deux segments (5, 6).

4. Électrode selon l'une des revendications 1 à 3, **caractérisée en ce que** ladite au moins une saillie (2b) est réalisée sous forme d'une pointe s'amincissant dans une direction opposée à la zone de retenue (2e).

5. Électrode selon l'une des revendications 1 à 4, **caractérisée en ce que** l'élément de raccordement (2) comporte au moins deux segments d'aile (9), lesdits au moins deux segments d'aile (9) présentant une portion inclinée (9a) par rapport à une position horizontale (H), les segments d'aile formant la saillie (2b) et la zone de retenue en forme de bride (2e) s'écartant latéralement, lesdits au moins deux segments d'aile étant de préférence au moins partiellement réalisés avec des arêtes vives.

6. Électrode selon l'une des revendications 1 à 5, **caractérisée en ce que** le conducteur (3) est réalisé sous forme de disque conducteur dépassant au moins partiellement au-delà de la zone élargie (BZ) déformée.

7. Électrode selon l'une des revendications 1 à 6, **caractérisée en ce que** l'élément de raccordement (2), d'une part, et le milieu de contact (4), d'autre part, sont disposés sur le support (1) à des emplacements décalés latéralement l'un par rapport à l'autre.

8. Électrode selon l'une des revendications 1 à 7, **caractérisée en ce que** le support (1) est revêtu, sur la face orientée vers la peau, d'un adhésif, de préférence un adhésif cutané, lequel adhésif est de préférence auto-adhésif ou activable thermiquement, ou comporte une couche de type pansement (7) munie d'un adhésif, de préférence un adhésif cutané.

9. Électrode selon l'une des revendications 1 à 8, **caractérisée en ce que** le conducteur (3) et le milieu de contact (4) sont constitués d'éléments distincts et de préférence de matériaux différents (fig. 1 à 17b), ou **en ce que** le conducteur (3) et le milieu de contact (4) sont constitués d'un seul et même élément, de préférence une colle électriquement conductrice au moyen de laquelle l'électrode peut être fixée sur la peau d'un patient (fig. 18 à 23).

10. Électrode selon l'une des revendications 1 à 9, **caractérisée en ce que** l'élément de raccordement (2) et/ou le milieu de contact (4) sont constitués au moins partiellement d'un couple argent/chlorure d'argent, étain/chlorure d'étain ou d'un autre couple rédox approprié par exemple pour la dépolarisation d'une électrode, ou contiennent un tel couple.

11. Procédé de fabrication d'une électrode destinée à être appliquée sur la peau humaine selon l'une des revendications 1 à 10, **caractérisé par** les étapes consistant à :
- disposer, de préférence par collage ou impression, un conducteur (3) sur la face inférieure dirigée vers la peau d'un support (1) électriquement non conducteur, le conducteur (3) étant revêtu sur une face d'un matériau électriquement conducteur, le matériau électriquement conducteur étant constitué d'un couple argent/chlorure d'argent, étain/chlorure d'étain ou d'un autre couple rédox approprié par exemple pour la dépolarisation d'une électrode, ou contenant un tel couple,
- introduire un élément de raccordement (2) depuis la face supérieure du support (1) à travers celui-ci, de sorte que la saillie (2b) de l'élément de raccordement (2) dépasse sur la face inférieure opposée du support (1) et que l'élément de raccordement (2), de préférence muni d'une zone de retenue en forme d'assiette (2e) s'écartant latéralement, repose sur la face supérieure du support (1), l'élément de raccordement (2) étant relié au support (1) sur la face inférieure et la face supérieure de celui-ci avec interposition du conducteur de forme plane,
- déformer la saillie (2b) de l'élément de raccordement (2) de manière à former une zone élargie (BZ) déformée permettant d'une part d'établir une connexion électrique entre l'élément de raccordement (2) et le conducteur (3) et d'autre part d'assurer une fixation mécanique de l'élément de raccordement (2) sur le support (1).

12. Procédé selon la revendication 11, **caractérisé en ce que** la déformation de la saillie (2b) est réalisée par :
- fusion de la saillie (2b), et/ou
- sertissage de la saillie (2b), et/ou
- évasement de la saillie (2b), et/ou
- pliage de la saillie (2b).
